# EUROPEAN PATENT APPLICATION

(11) **EP 1 815 831 A1**
(43) Date of publication of application: **08.08.2007**
(21) Application number: 07001534.2
(22) Date of filing: 24.01.2007
(51) Int. Cl.: A61F 13/15

(54) **Disposal absorbent product**

(30) Priority: 02.02.2006 JP 2006026212
(71) Applicant: Livedo Corporation, Shikokuchuo-shi, Ehime 799-0122 (JP)
(72) Inventor: Takahashi, Yuki Tokushima Sadamitsu Plant, Tsurugi-cho Mima-gun Takushima 779-4104 (JP); Miyake, Hirofumi Tokushima Sadamitsu Plant, Tsurugi-cho Mima-gun Takushima 779-4104 (JP)
(74) Representative: Müller-Boré & Partner Patentanwälte

(57) **Abstract**

An absorbent product (1) is a pants-type disposal diaper and comprises a first mark (51) and a second mark (52) which are provided on a front part (201) and a back part (203) of a body part (2) and visually recognizable from the outside of the body part (2). The first mark (51) and the second mark (52) include information for discriminating the front and back of the body part (2). The whole of the first mark (51) and the second mark (52) are provided in an area on one side of a body part center line (20) along vertical direction, with the area overlapping with an absorbent core (22). This makes it possible to suppress conspicuousness of the first mark (51) and the second mark (52) in the front part (201) and the back part (203) and reduce shame of wearer of the absorbent product (1).

## Description

### BACKGROUND OF THE INVENTION

### Field of the Invention

The present invention relates to a pants-type disposal absorbent product which has a waist opening at an upper end and a pair of leg openings on a lower part.

### Description of the Background Art

Conventionally, a pants-type has been used as a type of disposal diapers for receiving excrement from a wearer. There are some pants-type disposal diapers where letters such as "FRONT" or "BACK" are printed on a front part or back part of a body part so that the front and back of a disposal diaper can be easily discriminated in wearing.

Japanese Patent Application Laid-Open No. 2005-74157 discloses a technique for easily confirming letters representing size of a disposal diaper even if a plurality of disposal diapers are overlapped, where the letters are printed in the vicinity of a crotch part of an outer covering sheet in a width direction (i.e., horizontal direction) in a line.

Meanwhile, since an appearance of pants-type disposal diaper is similar to those of closing such as a normal pants, a wearer (especially, the aged) tends to feel shame in wearing a disposal diaper in which such letters or the like are largely printed on the center portion of the front part or the back part. However, if no discrimination letters or the like representing the front and back, size or the like are used, convenience of the wearer is greatly reduced in wearing a disposal diaper.

### SUMMARY OF THE INVENTION

The present invention is intended for a pants-type disposal absorbent product which has a waist opening at an upper end and a pair of leg openings on a lower part. It is an object of the present invention to reduce shame of a wearer of the pants-type disposal absorbent product.

The pants-type disposal absorbent product which has a waist opening at an upper end and a pair of leg openings on a lower part comprises a pants-like body part having a front part to be located on a stomach side of a wearer, a back part to be located on a back side of the wearer and a middle part to come into contact with a crotch region of the wearer; and a mark which is provided on at least one of the front part and the back part and is visually recognizable from the outside of the body part, a center position of the mark being apart from a center line of the body part which extends from the front part through the middle part to the back part. Since the center position of the mark is apart from the center line of the body part in the present invention, it is possible to reduce shame of the wearer.

According to a preferred embodiment of the present invention, the body part comprises an absorbent core; a top sheet covering an inner side of the absorbent core, the inner side being opposite to the wearer; and a back sheet covering an outer side of the absorbent core, and in the disposal absorbent product, the mark is provided in an area overlapping with the absorbent core in the at least one of the front part and the back part. This makes it possible to easily recognize the mark.

According to another preferred embodiment of the present invention, the disposal absorbent product further comprises a waist elastic member which is adhered to the body part along an edge of a waist opening and is contracted to form waist opening gathers; leg elastic members which are adhered to the body part along edges of a pair of leg openings, respectively, and are contracted to form leg opening gathers; and front and back elastic members which are adhered to the front part and the back part along a horizontal direction between the waist elastic member and the leg elastic members and are contracted to form front part gathers and back part gathers, and in the disposal absorbent product, the mark is provided in an area other than areas where the front and back elastic members are adhered, in the at least one of the front part and the back part.

According to still another preferred embodiment of the present invention, the mark is apart from the center line of the body part.

According to an aspect of the present invention, the mark is used for discrimination of a front and a back of the body part.

According to another aspect of the present invention, the mark is provided on each of the front part and the back part of the body part.

These and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a view showing an appearance of an absorbent product;
Fig. 2 is a front view of the absorbent product;
Fig. 3 is a rear view of the absorbent product;
Fig. 4 is a plan view of the absorbent product in a state where the disposal absorbent product is spread;
Fig. 5 is a cross-sectional view of the absorbent product;
Figs. 6 and 7 are front views showing other examples of the absorbent product; and
Figs. 8 and 9 are a front view and a rear view showing still another example of the absorbent product, respectively.

### DESCRIPTION OF THE PREFERRED EMBODIMENTS

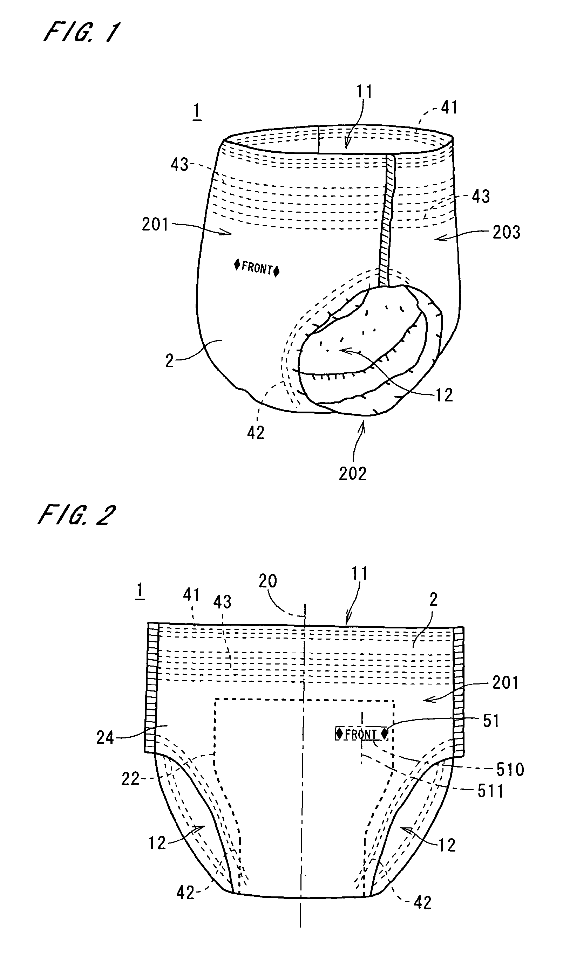

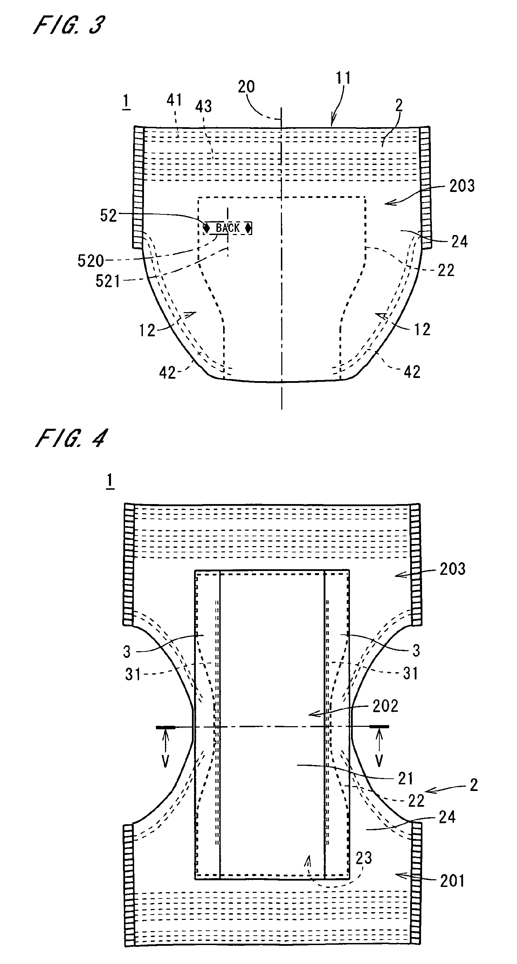

Fig. 1 is a view showing an appearance of a disposal absorbent product 1 in accordance with a preferred embodiment of the present invention. Fig. 2 is a front view (i.e., view of a portion located on a stomach side (an abdomen) of a wearer) of the disposal absorbent product 1 and Fig. 3 is a rear view (i.e., view of a portion located on a back side (a back) of the wearer) of the disposal absorbent product 1. The disposal absorbent product 1 is a pants-type (i.e., pull-up type) disposal diaper which has a waist opening 11 at an upper end (i.e., an end on the upper side of Fig. 1) and a pair of leg openings 12 on a lower part.

Fig. 4 is a plan view of the disposal absorbent product 1 as viewed from the wearer's side in a state where the disposal absorbent product 1 is spread. As shown in Fig. 4, the disposal absorbent product 1 comprises a pants-like body part 2. In the disposal absorbent product 1, a lower portion 201 in Fig. 4 of the body part 2 is located on the stomach side of the wearer, and an upper portion 203 shown in Fig. 4 is located on the back side of the wearer. In the following description, the portions 201 and 203 which are located on the stomach side and the back side of the wearer in the body part 2 are referred to as a "front part 201" and a "back part 203", respectively, and a portion 202 to come into contact with a crotch region of the wearer between the front part 201 and the back part 203 is referred to as a "middle part 202".

As shown in Fig. 1, in the disposal absorbent product 1, the body part 2 is folded in the middle part 202, and portions on the both sides in a horizontal direction of the front part 201 (i.e., portions on the both sides in a width direction of the front part 201) in a case where the middle part 202 faces downwards are adhered to portions on the both sides in the horizontal direction of the back part 203 (i.e., portions on the both sides in the width direction of the back part 203), respectively.

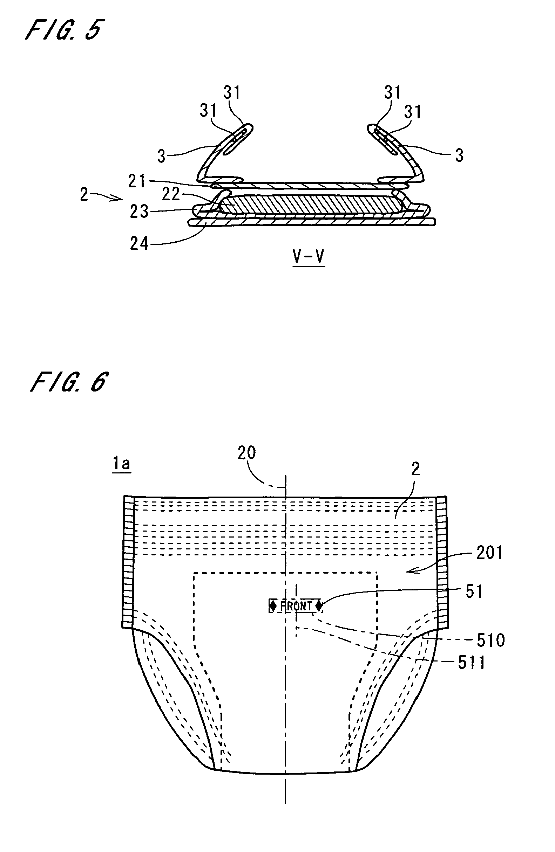

Fig. 5 is a cross-sectional view of the disposal absorbent product 1 taken along a line V-V in Fig. 4. As shown in Figs. 4 and 5, the body part 2 comprises an absorbent core 22, a top sheet 21 covering a main surface of an inner side of the absorbent core 22 (on the side of wearer), a back sheet 23 covering a main surface of an outer side of the absorbent core 22 (opposite to the wearer side), an outer covering sheet 24 covering an outer side of the back sheet 23, and a pair of side wall parts 3 which are provided over an almost entire length in a longitudinal direction (i.e., a vertical direction in Fig. 4) of the absorbent core 22, on the both sides in the width direction perpendicular to the longitudinal direction of the absorbent core 22.

The contour of the absorbent core 22 is shown by a thick broken-line in Fig. 4. In the plan view, the absorbent core 22 has a form of hourglass, where a width in the horizontal direction of each of both ends in the longitudinal direction is greater than a width in the horizontal direction of the middle part 202. The top sheet 21 and the back sheet 23 are adhered around the absorbent core 22 each other by a hot melt adhesive or the like, and the side wall part 3 is adhered to each side of the absorbent core 22. The outer covering sheet 24 and the back sheet 23 are adhered by the hot melt adhesive or the like.

The top sheet 21 is a nonwoven fabric made of liquid-pervious material, for example, hydrophilic fiber, and the top sheet 21 immediately catches moisture of excrement from the wearer and moves the moisture into the absorbent core 22. Examples of the nonwoven fabric used for the top sheet 21 are a point-bond nonwoven fabric, air-through nonwoven fabric, or spunlace nonwoven fabric, and as hydrophilic fibers for making these nonwoven fabrics, normally, cellulose, rayon, cotton or the like are used. As the top sheet 21, a liquid-pervious nonwoven fabric made of hydrophobic fiber (for example, polypropylene, polyethylene, polyester, polyamide, or nylon) on which surface hydrophilic treatment is performed with a surfactant may be utilized or a microporous plastic film may be used.

The absorbent core 22 is formed by wrapping a mixture of hydrophilic fibers (e.g., crushed pulp fibers or cellulose fibers) and granulated absorbent polymers (e.g., SAP (Super Absorbent Polymer)) in a cover sheet such as a tissue paper or a liquid-pervious nonwoven fabric, and the absorbent core 22 rapidly absorbs and retains moisture passing through the top sheet 21. The cover sheet is adhered to the hydrophilic fibers and the absorbent polymers with the hot melt adhesive, whereby deformation of the hydrophilic fibers and fall of the absorbent polymers (especially, fall after absorption of moisture) are prevented.

The back sheet 23 is a water-repellent or liquid-impervious plastic film, and the back sheet 23 prevents the moisture which has passed through the top sheet 21 and the moisture which is retained in the absorbent core 22 from leaking out into the outer covering sheet 24. From the view point of comfort for wearer, it is preferable that a plastic film with breathability is used as the back sheet 23. As the back sheet 23, a water-repellent or liquid-impervious nonwoven fabric or a laminated sheet in which a water-repellent or liquid-impervious plastic film is laminated on an inner side of a water-repellent or liquid-impervious nonwoven fabric can be used. Nonwoven fabrics used for the back sheet 23 are, for example, a spunbond nonwoven fabric, a meltblown nonwoven fabric, or a SMS (spunbond-meltblown-spunbond) nonwoven fabric, on which water-repellent treatment may be applied as necessary.

The outer covering sheet 24 is a water-repellent or liquid-impervious nonwoven fabric, and portions on the both sides of the outer covering sheet 24 in the front part 201 are adhered to portions on the both sides of the outer covering sheet 24 in the back part 203, respectively, to form the absorbent product 1 in the form of pants. As the outer covering sheet 24, a water-repellent or liquid-impervious plastic film or a laminated sheet of plastic film and nonwoven fabric may be utilized, however, it is preferable that the outer covering sheet 24 is formed by nonwoven fabric from the view point of improving the feel of the absorbent product 1.

As shown in Figs. 4 and 5, two elastic yarns 31 extending in the longitudinal direction are adhered to each of the pair of side wall parts 3 and in the expanded absorbent product 1 shown in Fig. 4, the elastic yarns 31 are extended. In the absorbent product 1, by contracting the elastic yarns 31, the side wall parts 3 stand up toward the wearer in the both sides of the body part 2 to form standing gathers which come into contact with the vicinity of wearer's crotch in wear.

The side wall parts 3 are made of water-repellent or liquid-impervious nonwoven fabric (i.e., spunbond nonwoven fabric, meltblown nonwoven fabric, or SMS nonwoven fabric), plastic film, or combinations of these materials. From the viewpoint of improving comfort of the absorbent product 1, it is preferable that the side wall parts 3 have breathability. As the elastic yarns 31, polyurethane yarn, strip-like polyurethane film, yarn-like or strip-like natural rubber, or the like are used for example.

As shown in Figs. 1 to 3, the absorbent product 1 further comprises waist elastic members 41 which are adhered to the body part 2 along the edge of the waist opening 11 around the whole waist opening 11, leg elastic members 42 which are adhered to the body part 2 along each edge of the pair of leg openings 12 around the almost whole leg opening 12, and front and back elastic members 43 which are located between the waist elastic members 41 and the leg elastic members 42 and adhered to the front part 201 and the back part 203 along the horizontal direction (i.e., width direction).

The waist elastic members 41, the leg elastic members 42, and the front and back elastic members 43 have a plurality of elastic yarns, respectively. Examples of the elastic yarns include polyurethane yarn, strip-like polyurethane film, yarn-like or strip-like natural rubber or the like, and the expanded elastic yarns are adhered with the outer covering sheet 24 by the hot melt adhesive or the like. By contracting the elastic yarns, waist opening gathers and leg opening gathers are formed around the waist opening 11 and the leg openings 12, and front part gathers and back part gathers are formed across the front part 201 and the back part 203 of the body part 2. In the portions to which the front and back elastic members 43 are adhered, the outer covering sheet 24 has a structure where two nonwoven fabrics laminated, and the front and back elastic members 43 are adhered between the above two nonwoven fabrics. Accurately, the elastic yarns of the waist elastic members 41 and the front and back elastic members 43 are individually provided in the front part 201 and the back part 203.

In the absorbent product 1 which is worn by the wearer, a portion close to the waist opening 11 fits the wearer's waist by contracting the waist elastic members 41, and portions close to the leg openings 12 fit the wearer's legs by contracting the leg elastic members 42. The front part 201 and the back part 203 fit on the stomach side and the back side of the wearer by contracting the front and back elastic members 43. As discussed above, it is possible to prevent the absorbent product 1 from being loosened by the load of excrement or the like by improving fitting of the absorbent product 1 to the wearer.

In a state where the absorbent product 1 is worn by the wearer (i.e., state where the waist elastic members 41, the leg elastic members 42, and the front and back elastic members 43 are extended), a tension of each of the plurality of elastic yarns in the front and back elastic members 43 is smaller than that in the waist elastic members 41. Thus, it is possible to prevent the front part 201 and the back part 203 in the body part 2 from fitting too tightly to the wearer and to provide the wearer with comfortable feeling in wear of the absorbent product 1.

As shown in Fig. 2, the absorbent product 1 comprises a first mark 51 which is provided on the front part 201 and is visually recognizable from the outside of the body part 2 (i.e., the first mark 51 is provided opposite to the wearer side of the body part 2). In the first mark 51, a letter "FRONT" for discrimination of the front and back of the body part 2 in the absorbent product 1 is horizontally sandwiched between two rhombuses. In other words, the first mark 51 includes information for discriminating the front and back of the body part 2.

The first mark 51 is provided in an area on the right side of the center line 20 (i.e., on the left side as viewed from the wearer) which extends from the front part 201 through the middle part 202 to the back part 203 in the body part 2 (i.e., the center line 20 is a line along the vertical direction perpendicular to the horizontal direction in Fig. 2, and hereinafter referred to as "body part center line 20"), with the area overlapping with the absorbent core 22 and located under areas where the front and back elastic members 43 are adhered. The center line 511 along the vertical direction of the first mark 51 (hereinafter, referred to as "first mark center line 511") is parallel to the body part center line 20 and is apart from the body part center line 20 in the horizontal direction. The first mark center line 511 refers to a line which elongates in the vertical direction through the center in the horizontal direction of a virtual rectangular region 510 circumscribing the first mark 51 (in the preferred embodiment, the rectangular region 510 is a region which has the same lengths in the vertical and horizontal directions as the first mark 51 and surrounded by a two-dot chain line in Fig. 2). In the preferred embodiment, the lengths in the horizontal and vertical directions of the rectangular region 510 are about 5 cm and about 1.5 cm, respectively.

As shown in Fig. 3, the absorbent product 1 comprises a second mark 52 which is provided on the back part 203 and is visually recognizable from the outside of the body part 2. In the second mark 52, a letter "BACK" is horizontally sandwiched between two rhombuses, and like the first mark 51, the second mark 52 includes information for discriminating the front and back of the body part 2.

The second mark 52 is provided in an area on one side of the body part center line 20 (i.e., in the preferred embodiment, the one side is the left side in Fig. 3 and also the left side as viewed from the wearer), with the area overlapping with the absorbent core 22 and located under areas where the front and back elastic members 43 are adhered. The second mark center line 521 is parallel to the body part center line 20 and is apart from the body part center line 20 in the horizontal direction. Like the first mark center line 511, the second mark center line 521 refers to a line which elongates in the vertical direction through the center in the horizontal direction of a virtual rectangular region 520 (surrounded by a two-dot chain line in Fig. 3) circumscribing the second mark 52.

As shown in Figs. 2 and 3, the first mark 51 and the second mark 52 are arranged on the same side of the body part 2 with respect to the body part center line 20 (in the preferred embodiment, the above same side is the left side as viewed from the wearer). In the formation process of the body part 2 in the absorbent product 1, the first mark 51 and the second mark 52 are printed on the outer side of the outer covering sheet 24 (opposite to the wearer side of the outer covering sheet 24) in an inkjet printing.

In wearing the absorbent product 1, since the first mark 51 and the second mark 52 are visually recognized by a wearer, a caregiver or the like, the front and back of the absorbent product 1 can be easily discriminated. As a result, it is possible to easily put the absorbent product 1 on the wearer.

As discussed above, in the absorbent product 1, since the first mark center line 511 of the first mark 51 is apart from the body part center line 20 of the body part 2, in other words, since the center position of the first mark 51 is apart from the body part center line 20, it is possible to suppress conspicuousness of the first mark 51 in the front part 201 and reduce shame of the wearer (especially, the aged) of the absorbent product 1, in comparison with the case where the position of the first mark center line 511 is identical to that of the body part center line 20 (i.e., the case where the center of the first mark 51 is located at the center in the horizontal direction of the body part 2).

Similarly in the second mark 52, since the center position of the second mark 52 is apart from the body part center line 20, it is possible to suppress conspicuousness of the second mark 52 in the back part 203 and reduce shame of the wearer. In the absorbent product 1, the mark (i.e., the first mark 51 and the second mark 52) is provided on each of the front part 201 and the back part 203 of the body part 2, and the mark can be visually recognized from any side of the front and back of the absorbent product 1. In taking the absorbent product 1 or in the worn state of the absorbent product 1, a user (e.g., the caregiver who looks after wearing of the absorbent product 1 or the wearer itself) can confirm the mark easily regardless of directions of the absorbent product 1 and can discriminate the front and back of the absorbent product 1 easily. As a result, it is possible to more easily put the absorbent product 1 on the wearer.

In the absorbent product 1, since the first mark 51 is provided on only one side of the body part center line 20 with respect to the horizontal direction (i.e., the whole first mark 51 is apart from the body part center line 20), it is possible to more suppress conspicuousness of the first mark 51, and further reduce shame of the wearer (same as in the second mark 52). The first mark 51 and the second mark 52 are arranged on the same side of the body part center line 20 with respect to the horizontal direction (on the left side as viewed from the wearer in the preferred embodiment). Even if the wearer wrongly reads the discrimination information of the front and back shown by the first mark 51 and the second mark 52, the marks appear on the opposite side to the side in the correct worn state (i.e., the right side as viewed from the wearer) and therefore, the wearer can easily notice he/her wearing the absorbent product 1 in the wrong way and rewear the absorbent product 1. Additionally, the first mark 51 and the second mark 52 may be arranged on the right side as viewed from the wearer in the absorbent product 1 which is correctly worn by the wearer.

In the absorbent product 1, the first mark 51 and the second mark 52 are provided in the area overlapping with the absorbent core 22 (i.e., the area is thicker and hard to bend, in comparison with an area without overlapping with the absorbent core 22), and in the front part 201 and the back part 203, it is possible to suppress wrinkles of the areas where the first mark 51 and the second mark 52 are arranged. This makes it possible to easily recognize the first mark 51 and the second mark 52.

In the front part 201 and the back part 203 of the body part 2, since the first mark 51 and the second mark 52 are provided in an area other than the areas where the front and back elastic members 43 are adhered (i.e., the area is not substantially affected by wrinkles caused by contraction of the front and back elastic members 43 or the like), the first mark 51 and the second mark 52 can be recognized more easily.

Though the preferred embodiment of the present invention has been discussed above, the present invention is not limited to the above-discussed preferred embodiment, but allows various variations.

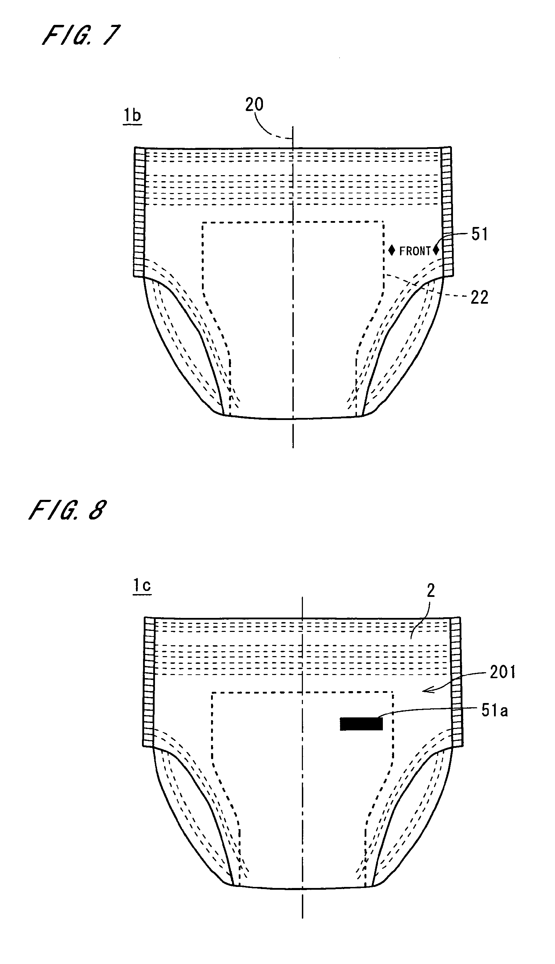

Like an absorbent product 1 a shown in Fig. 6, the first mark 51 may be arranged across the body part center line 20 (i.e., so that the rectangular region 510 surrounding the first mark 51 is located on the both sides of the body part center line 20), as long as the center position of the first mark 51 is apart from the body part center line 20 in the front part 201 of the body part 2 (i.e., the first mark center line 511 is apart from the body part center line 20). From the viewpoint of more suppressing conspicuousness of the first mark 51, it is preferable that the first mark 51 is largely away from the body part center line 20 with respect to the horizontal direction. In this case, the first mark 51 may be provided in an area without overlapping with the absorbent core 22, like an absorbent product 1 b shown in Fig. 7. The second mark 52 also has the above-discussed features of the first mark 51.

The first mark 51 and the second mark 52 do not necessarily have to be printed on the outer side of the outer covering sheet 24 (i.e., opposite to the wearer side of the outer covering sheet 24) of the body part 2, but may be printed, for example, on an inner side of the outer covering sheet 24, on the back sheet 23, or on the cover sheet wrapping the hydrophilic fibers or the like of the absorbent core 22. In this case, the first mark 51 and the second mark 52 are visually recognizable through the outer covering sheet 24.

The first mark 51 and the second mark 52 are not necessarily limited to a combination of alphabet and sign but can be consisted of only letters such as alphabet, hiragana, katakana or kanji. Further, the first mark 51 and the second mark 52 may be consisted of a sign or a pattern figure (for example, figures such as a character or animal) or may be a combination of letter, sign, pattern figure or the like. From the viewpoint of easily discriminating the front and back of the absorbent product 1, it is preferable that the first mark 51 and the second mark 52 include letters representing the front and back of the absorbent product 1. From the viewpoint of suppressing conspicuousness of the marks and reducing shame of a wearer, it is preferable that both of the first mark 51 and the second mark 52 are consisted of only figure or sign.

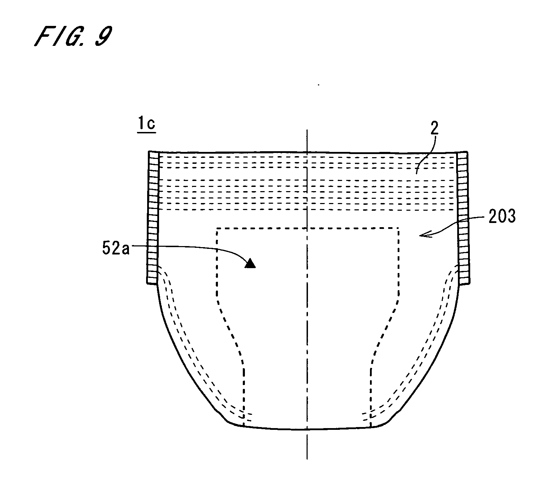

The first mark and the second mark do not necessarily have to include information for discrimination of the front and back in a form of letters, for example like an absorbent product 1c shown in Figs. 8 and 9, there may be a case where a rectangular first mark 51a and a triangular second mark 52a are provided on the front part 201 and the back part 203 of the body part 2, respectively, and the instructions "Please wear this absorbent product so that a rectangular mark is located on your stomach side and a triangular mark is on your back side." is mentioned in a package bag of the absorbent product 1 or the like. In this way, the first mark and the second mark may be used together with instructions or the like which are the information for discrimination of the front and back of the absorbent product 1. For clear distinction between the first mark and the second mark, colors of the both marks may be different from each other.

The first mark and the second mark can include information for discrimination of, e.g., size of the absorbent product 1 or gender of a wearer, as well as the information for discrimination of the front and back. The first mark and the second mark may be a trade name, a logo or the like which represent a manufacturer, a selling agency or the like of the absorbent product or may be marks for mere decoration without including special information.

In the absorbent product 1, the mark does not necessarily have to be provided on each of the front part 201 and the back part 203 but may be provided on at least one of the front part 201 and the back part 203. In other words, one of the first mark 51 and the second mark 52 may be provided. When only the first mark 51 is provided, also in the course of wearing the absorbent product 1 (for example, at the stage where both legs have just been putted into the absorbent product 1), it is possible to visually recognize the first mark 51 easily and immediately discriminate the front and back of the absorbent product 1. When only the second mark 52 is provided, it is rare that the second mark 52 comes into a wearer's sight after wearing and therefore, shame of the wearer can be more reduced.

In the absorbent product 1, there may be a case where portions on the both sides in the horizontal direction (i.e., the both sides in the width direction) of the top sheet 21 and the back sheet 23 in the front part 201 are directly adhered to portions on the both sides of the top sheet 21 and the back sheet 23 in the back part 203, respectively, without using the outer covering sheet 24, to form the pants-like body part 2. In this case, the outer covering sheet 24 can be omitted. When omitting the outer covering sheet 24, the first mark 51 and the second mark 52 are printed on an outer side of the back sheet 23 or the like so as to be visually recognizable from the outside of the back sheet 23.

While the invention has been shown and described in detail, the foregoing description is in all aspects illustrative and not restrictive. It is therefore understood that numerous modifications and variations can be devised without departing from the scope of the invention.

## Claims

1. A pants-type disposal absorbent product (1) which has a waist opening (11) at an upper end and a pair of leg openings (12) on a lower part, comprising:
a pants-like body part (2) having a front part (201) to be located on a stomach side of a wearer, a back part (203) to be located on a back side of said wearer and a middle part (202) to come into contact with a crotch region of said wearer; and
a mark (51, 52) which is provided on at least one of said front part (201) and said back part (203) and is visually recognizable from the outside of said body part (2), and said disposal absorbent product **characterized in that**
a center position of said mark (51, 52) is apart from a center line (20) of said body part (2) which extends from said front part (201) through said middle part (202) to said back part (203).

2. The disposal absorbent product according to claim 1, wherein
said body part (2) comprises:
an absorbent core (22);
a top sheet (21) covering an inner side of said absorbent core (22), said inner side being opposite to said wearer; and
a back sheet (23) covering an outer side of said absorbent core (22), wherein
said mark (51, 52) is provided in an area overlapping with said absorbent core (22) in said at least one of said front part (201) and said back part (203).

3. The disposal absorbent product according to claims 1 or 2, further comprising:
a waist elastic member (41) which is adhered to said body part (2) along an edge of a waist opening (11) and is contracted to form waist opening gathers;
leg elastic members (42) which are adhered to said body part (2) along edges of a pair of leg openings (12), respectively, and are contracted to form leg opening gathers; and
front and back elastic members (43) which are adhered to said front part (201) and said back part (203) along a horizontal direction between said waist elastic member (41) and said leg elastic members (42) and are contracted to form front part gathers and back part gathers, and said disposal absorbent product **characterized in that**
said mark (51, 52) is provided in an area other than areas where said front and back elastic members (43) are adhered, in said at least one of said front part (201) and said back part (203).

4. The disposal absorbent product according to any one of claims 1 to 3, wherein
said mark (51, 52) is apart from said center line (20) of said body part (2).

5. The disposal absorbent product according to any one of claims 1 to 4, wherein
said mark (51, 52) is used for discrimination of a front and a back of said body part (2).

6. The disposal absorbent product according to any one of claims 1 to 5, wherein
said mark (51, 52) is provided on each of said front part (201) and said back part (203) of said body part (2).
